# EUROPÄISCHE PATENTSCHRIFT

(11) **EP 3 117 868 B1**
(45) Veröffentlichungstag und Bekanntmachung des Hinweises auf die Patenterteilung: **01.11.2023**
(21) Anmeldenummer: 15002121.0
(22) Anmeldetag: 16.07.2015
(51) Int. Cl.: A61M 39/10, A61M 1/00, F16L 19/065, A61M 39/12

(54) **ANSCHLUSSVORRICHTUNG FÜR DIE WUNDVERSORGUNG UND WUNDVERSORGUNGSKIT**
CONNECTION DEVICE FOR WOUND CARE AND WOUND CARE KIT
DISPOSITIF DE RACCORDEMENT POUR LE TRAITEMENT DE PLAIES ET KIT DE TRAITEMENT DE PLAIES

(43) Veröffentlichungstag der Anmeldung: 18.01.2017
(73) Patentinhaber: Lohmann & Rauscher GmbH, 2525 Schönau/Triesting (AT)
(72) Erfinder: Hofstetter, Christoph, 1110 Wien (AT); Herzele, Arno, 2345 Brunn am Gebirge (AT)
(74) Vertreter: Seranski, Klaus

(56) Entgegenhaltungen:
- EP-A1- 2 233 814
- EP-A2- 1 398 556
- WO-A2-03/057070
- DE-A1-102008 008 332
- DE-A1-102008 021 312
- US-A- 4 754 782
- US-A1- 2008 009 832
- US-A1- 2008 011 368
- US-B1- 7 678 102

## Beschreibung

Die Erfindung betrifft eine Vorrichtung zum Anschließen von mehrlumigen Leitungen nach dem Oberbegriff des Patentanspruchs 1 im medizinischen Bereich, beispielsweise für die Wundversorgung, insbesondere im Bereich der Unterdrucktherapie, mit einem zum dichten und vorzugsweise lösbaren Anschließen von mindestens zwei Leitungslumen ausgelegten Grundkörper sowie ein Behandlungskit für die Unterdrucktherapie mit mindestens einer erfindungsgemäßen Anschlussvorrichtung und mindestens einer daran anschließbaren mehrlumigen Leitung.

Bei der Unterdrucktherapie wird im Bereich einer zu behandelnden Wunde ein Unterdruck erzeugt, um so den Wundheilungsprozess zu fördern. Dazu wird die Wundregion mit einem Schlauch an eine entsprechende Pumpe angeschlossen, mit der gleichzeitig Exsudat aus der Wundregion abgesaugt werden kann. Zusätzlich kann es erforderlich sein, den Druck in der Wundregion über ein gesondertes Leitungslumen zu messen, die Wundregion über gesonderte Leitungslumen zu belüften, Wundreinigungsmittel, Medikamente o. ä. zuzuführen etc. Bei einzelnen Indikationen kann es vorteilhaft sein, im Bereich der Wundregion eine kontinuierliche Strömung aufrechtzuerhalten. Bei anderen Indikationen kann es sinnvoll sein, einen konstanten Unterdruck aufrechtzuerhalten. Schließlich ist es bei einigen Indikationen auch sinnvoll, den Unterdruck in einer vorgegebenen zeitlichen Abfolge zu erzeugen und wieder zu reduzieren, das heißt einen intermittierenden Unterdruck im Bereich der Wundregion anzulegen.

Dabei hat es sich als vorteilhaft erwiesen, wenn zur Belüftung zwei, drei oder mehr Leitungslumen eingesetzt werden, während der Unterdruck über ein Leitungslumen mit größerem Leitungsquerschnitt erzeugt werden kann.

Zum Zweck der Unterdrucktherapie müssen die Leitungslumen mit einem geeigneten Unterdrucktherapiegerät verbunden werden. Dabei ist darauf zu achten, daß die Verbindung einerseits fest halten soll, sich andererseits bei Bedarf manuell/kontrolliert leicht, rasch und unkompliziert öffnen lässt.

Mit dem Unterdrucktherapiegerät wird einerseits der Unterdruck erzeugt und andererseits ggf. eine Belüftung bzw. Belüftung und/oder Druckmessung der Wundregion und/oder andere der vorstehend beschriebenen Aufgaben ausgeführt. Darüber hinaus ist es in vielen Fällen erforderlich, zwei-, drei- oder mehrlumige Leitungen miteinander zu verbinden, wenn sich beispielsweise die Position des Unterdrucktherapiegeräts in Relation zum Patienten ändert und die Länge der Leitung entsprechend angepasst werden muss. Bereits vorhandene Leitungen können dabei weiterbenutzt werden, wenn Verlängerungsleitungen mit geeigneten Vorrichtungen dicht daran angeschlossen werden.

In der EP 2 240 233 B1 ist eine zweiteilige Verbindung bzw. Anschlussvorrichtung für mehrlumige Leitungen beschrieben, bei denen die einzelnen Lumen der Leitung in Form von einzelnen Schläuchen verwirklicht sind. Bei dieser Vorrichtung werden die einzelnen Schläuche einer mehrlumigen Leitung mit einer Vielzahl von Innenschlauchverbindungssteckerelementen in einem Innenverbindungsteilabschnitt abdichtend in Eingriff gebracht, wobei die einzelnen Innenschlauchverbindungssteckerelemente mit Außenschlauchverbindungszapfenelementen in Eingriff gebracht werden, an die die einzelnen Schläuche der anderen mehrlumigen Leitung angeschlossen sind. Bei diesen Anschlussvorrichtungen müssen zwei Verbindungsstückabschnitte vom Anwender dicht miteinander sowie jeweils mit den einzelnen Schläuchen der mehrlumigen Leitungen verbunden werden.

Eine Anschlussvorrichtung für einen Multilumen-Schlauch, bei dem sich die Lumen im Inneren der Leitung/des Schlauches befinden, ist in der EP 2 240 234 B1 beschrieben. Diese bekannte Vorrichtung ist zweiteilig und beinhaltet einen einzusteckenden Verbindungsstückabschnitt sowie einen aufnehmenden Verbindungsstückabschnitt, die jeweils eine Vielzahl von Strömungskanälen aufweisen, die der Anzahl und den Ausmaßen/dem Design (rund, oval, Durchmesser...) der in der Leitung vorhandenen Lumen exakt entsprechen müssen. In der EP 2 536 448 B1 ist ein Kopplungsteil für getrennte Schläuche beschrieben, das eine Vorrichtung aufweist, die eine Fluidzufuhr von einem Servicekanal in einen Drainagekanal ermöglicht, aber den umgekehrten Weg verhindert.

In der WO 2010/127461 A1 ist eine zweiteilige Kopplungsanordnung beschrieben, mit der mehrlumige (bevorzugt solche, die eine zentrale Drainageleitung sowie drei um diese Drainageleitung angeordnete Zusatzleitungen beinhalten) Schläuche miteinander verbunden werden können. Dabei können Drainageleitung sowie die Zusatzleitungen in einen gemeinsamen Hohlraum münden, um dort den Druck zu messen. Bei einer Anschlusseinrichtung gemäß US 8,002,313 B2 ist ein Hohlraum vorgesehen, in welchen die Lumen eines ersten und zweiten Schlauchs miteinander kommunizieren können, auch wenn die Schläuche radial versetzt zueinander angebracht sind.

Weiter ist es Stand der Technik, mehrlumige Leitungen in Form von Schläuchen zu verwirklichen, die von mehreren Lumen in axialer Richtung durchsetzt sind. In diesem Fall ist es nicht mehr erforderlich, einzelne Schläuche miteinander zu verbinden. Vielmehr reicht es aus, wenn ein mehrlumiger Schlauch angeschlossen wird. Bei diesen bekannten mehrlumigen Schläuchen kann ein zentrales Lumen vorgesehen sein, welches koaxial zur Schlauchachse verläuft. Weitere Lumen des Schlauches können radial versetzt angeordnet sein und durchsetzen den das zentrale Lumen begrenzenden Schlauchmantel in axialer Richtung parallel zur Schlauchachse. Besonders vorteilhaft ist eine ungerade Anzahl von Zusatzlumen, besonders bevorzugt drei Zusatzlumen. Dabei können die radial versetzt angeordneten Zusatzlumen mit geringerem Querschnitt ausgestattet sein als das zentrale Lumen. Es können zwei, drei oder mehr in Umfangsrichtung versetzt angeordnete radial angeordnete Zusatzlumen vorgesehen sein. Beim Anschluss entsprechender mehrlumiger Schläuche, insbesondere bei der Verbindung solcher mehrlumigen Schläuche, muss darauf geachtet werden, daß die einzelnen Lumen des einen Schlauchs in fluiddurchlässiger Verbindung mit den entsprechenden Lumen des anderen Schlauchs gelangen, ohne daß es zu einer unerwünschten Verbindung zwischen solchen Schlauchlumen kommt, die unterschiedlichen Zwecken dienen. Es muss beispielsweise in vielen Fällen darauf geachtet werden, daß das zur Erzeugung des Unterdrucks eingesetzte zentrale Lumen nicht in Verbindung mit den radial versetzt angeordneten Zusatzlumen gelangt, wobei gleichzeitig die radial versetzt angeordneten Zusatzlumen der miteinander zu verbindenden mehrlumigen Schläuche miteinander verbunden werden müssen.

Diese Verbindung erweist sich in vielen Fällen als problematisch, weil sie eine lagerichtige Anordnung der einzelnen Schlauchlumen zueinander erfordert, insbesondere der radial versetzt angeordneten Zusatzlumen, die exzentrisch, das heißt nicht konzentrisch, zur Schlauchachse verlaufen. Dabei können die einzelnen radial versetzt angeordneten Zusatzlumen auf einer konzentrisch zur Schlauchachse angeordneten Kreislinie des Schlauchmantels angeordnet sein.

Eine Vorrichtung nach dem Oberbegriff des Patentanspruchs 1 ist in der US 2008/0011368 A1 und der WO 03/057070 A2, Fig. 18 und 19, beschrieben.

Angesichts dieser Probleme im Stand der Technik liegt der Erfindung die Aufgabe zugrunde, eine Anschlussvorrichtung für mehrlumige Leitungen für die Wundversorgung, insbesondere im Bereich der Unterdrucktherapie, bereitzustellen, welche einen einfachen Anschluss der Leitungen unter zuverlässiger Sicherstellung der gewünschten Trennung einzelner Leitungslumen ermöglicht.

Die Erfindung umfasst eine Vorrichtung gemäß Anspruch 1. Vorteilhafte Ausführungsformen der Erfindung sind in den abhängigen Ansprüchen angegeben.

Nachstehend wird die Erfindung unter Bezugnahme auf die Zeichnung, auf die hinsichtlich aller erfindungswesentlichen und in der Beschreibung nicht näher herausgestellten Einzelheiten verwiesen wird, erläutert. In der Zeichnung zeigt:
- Fig. 1: eine schematische Darstellung eines mehrlumigen Schlauchs für ein erfindungsgemäßes Behandlungskit für die Unterdrucktherapie,
- Fig. 2: eine Darstellung einer ersten Ausführungsform einer erfindungsgemäßen Anschlussvorrichtung,
- Fig. 3: eine schematische Detaildarstellung einer erfindungsgemäßen Anschlussvorrichtung,
- Fig. 4: Dichtungsanordnungen für eine erfindungsgemäße Anschlussvorrichtung,
- Fig. 5: Sicherungseinrichtungen für erfindungsgemäße Anschlussvorrichtungen
- Fig. 6: eine weitere Ausführungsform einer erfindungsgemäßen Anschlussvorrichtung
- Fig. 7: eine Ausführungsform einer Dichthülse

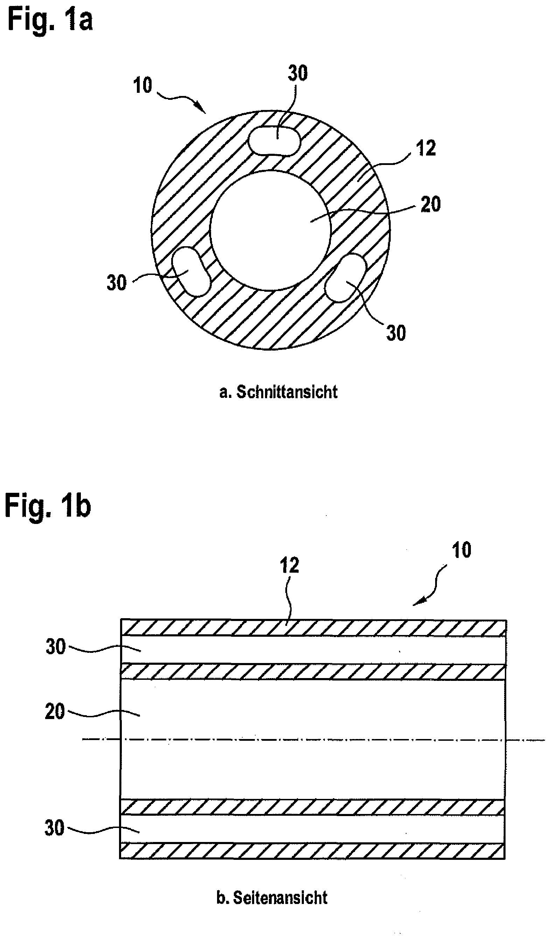

Fig. 1a zeigt eine Radialschnittdarstellung eines für ein erfindungsgemäßes Kit einsetzbaren mehrlumigen Schlauchs. Fig. 1b zeigt eine Axialschnittdarstellung des Schlauchs gemäß Fig. 1a. Der insgesamt mit 10 bezeichnete, eine mehrlumige Leitung bildende mehrlumige Schlauch weist ein von einem Schlauchmantel 12 umlaufenes zentrales Lumen 20 auf. Der Schlauchmantel 12 ist von einer Anzahl radial versetzt bezüglich dem zentralen Lumen 20 angeordneten Zusatzlumen 30 durchsetzt. Die Zusatzlumen sind in Umfangsrichtung gleichmäßig versetzt gegeneinander angeordnet. Sie weisen eine geringere Querschnittsfläche auf als das zentrale Lumen 20. Die Zentren der radial versetzt angeordneten Zusatzlumen 30 sind auf einer koaxial zur Schlauchachse verlaufenden Kreislinie angeordnet.

Bei erfindungsgemäßen Kits kann das zentrale Lumen 20 zum Erzeugen von Unterdruck im Wundbereich und zum Absaugen von Exsudat aus dem Wundbereich benutzt werden, während die radial versetzt angeordneten Zusatzlumen 30 z. B. zum Belüften der Wundregion zur Druckmessung und/oder zur Zufuhr von Medikation, Reinigungsflüssigkeiten etc. eingesetzt werden können. Das zentrale Lumen 20 einerseits und die radial versetzt angeordneten Lumen 30 andererseits müssen zumindest auf der Strecke zwischen Vakuumtherapiegerät und Wundregion voneinander getrennt sein.

Bei erfindungsgemäß bevorzugten mehrlumigen Schläuchen ist ein zentrales Lumen 20 und eine ungerade Anzahl symmetrisch angeordneter, radial versetzt bezüglich dem zentralen Lumen 20 angeordneter Zusatzlumen 30 vorgesehen. Dadurch wird eine besonders sichere Verbindung der Wunde mit dem Vakuumtherapiegerät gewährleistet, da sich durch diese Geometrie auch bei Klemmen oder Abknicken des Schlauchs zumindest eines der Zusatzlumen lediglich minimal verformt und somit beispielsweise bei einer Druckmessung kein Informationsverlust auftritt und auch kein Verbindungsabbruch (beispielsweise bei Belüftung der Wunde) befürchtet werden muss.

Als besonders vorteilhaft hat sich die in Fig. 1 dargestellte Schlauchgeometrie mit drei symmetrisch angeordneten radial versetzt angeordneten Zusatzlumen erwiesen. Bei dieser Geometrie können bei Abknicken nur ein bis zwei von drei Lumen signifikant verformt werden und auch die Scherkräfte, die beim Klemmen und Knicken im Inneren des Schlauchs wirken, haben bei dieser Geometrie den geringsten Einfluss auf die Durchlässigkeit der Lumen.

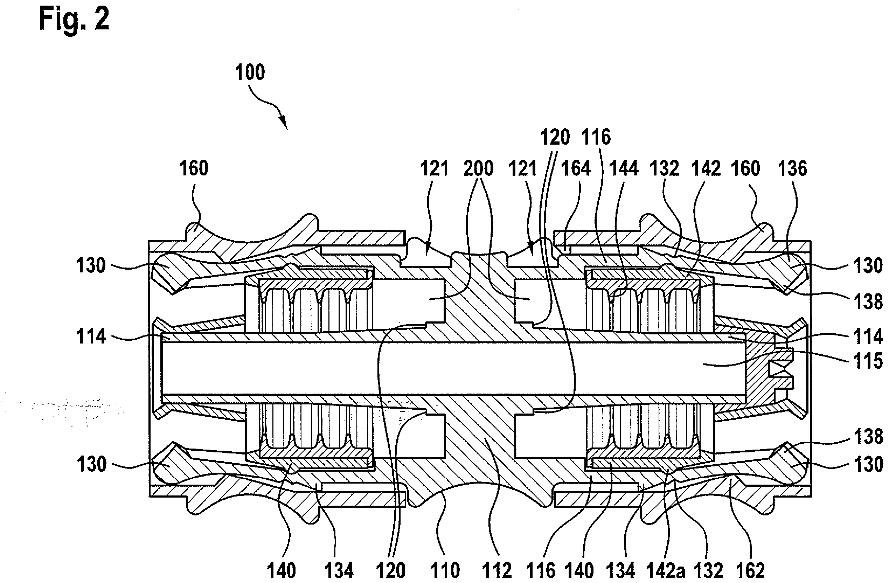

Die in Fig. 2 dargestellte Anschlussvorrichtung ist zum Verbinden von zwei mehrlumigen Schläuchen der in Fig. 1 dargestellten Art ausgelegt. Die insgesamt mit 100 bezeichnete Anschlussvorrichtung umfasst einen Grundkörper 110, zwei Dichthülsen 140 sowie zwei Klemmhülsen 160.

Der Grundkörper 110 weist einen zentralen Bund 112 auf, von dem ausgehend sich zwei Anschlussstutzen 114 koaxial zueinander in einander entgegengesetzte Richtungen erstrecken. Die Anschlussstutzen 114 sind von einer durchgehenden Bohrung 115 durchsetzt. Der Bund 112 umläuft die Anschlussstutzen 114 vollständig. In einem radialen Abstand von den Anschlussstutzen 114 sind Kammerhülsen 116 an den Bund 112 angesetzt. Die Kammerhülsen 116 erstrecken sich koaxial zueinander und koaxial zu den Anschlussstutzen 114 in einander entgegengesetzter Richtung ausgehend vom Bund 112. Zwischen den Kammerhülsen 116 und den Anschlussstutzen 114 sind Ringspalte gebildet, in die der Mantel 12 des mehrlumigen Schlauchs 10 eingeführt werden kann. Dabei wird die Einführtiefe des Schlauchs in den Ringspalt durch Anschläge 120 begrenzt, die als radial erweiterte Bereiche der Anschlussstutzen 114 an deren dem Bund 112 zugewandten Enden ausgeführt sind. Wie bei 121 angedeutet, sind die Kammerhülsen im Bereich des Bunds bzw. der Anschläge 112 mit Fenstern ausgestattet, welche eine Sichtkontrolle der Anlage der Stirnflächen des Schlauchs 10 an den Anschlägen 120 ermöglichen.

In die zwischen den Anschlussstutzen 114 und den Kammerhülsen 116 gebildeten Ringspalte sind die Dichthülsen 140 eingesetzt, so daß die äußeren Begrenzungsflächen der Dichthülsen mit Hilfe einer Ringschnappverbindung formschlüssig an der inneren Begrenzungsfläche der Kammerhülsen anliegen. Die Dichthülsen 140 umfassen einen starren bzw. nicht verformbaren Hülsenkörper 142, beispielsweise aus Polypropylen, und eine Mehrzahl von in axialem Abstand voneinander angeordneten verformbaren Dichtlamellen 144, die bei eingeschobener Leitung 10 an der äußeren Begrenzungsfläche der Leitung anliegen und so eine Mehrkammerdichtung bilden. Bei anderen Ausführungsformen der Erfindung kann nur eine Dichtlamelle vorgesehen sein. An der äußeren Begrenzungsfläche der starren Dichthülse 142 ist eine umlaufende Wulst 142a gebildet, die zur Bildung der Ringschnappverbindung in einer radial innenliegenden Vertiefung der Kammerhülse 116 aufgenommen ist.

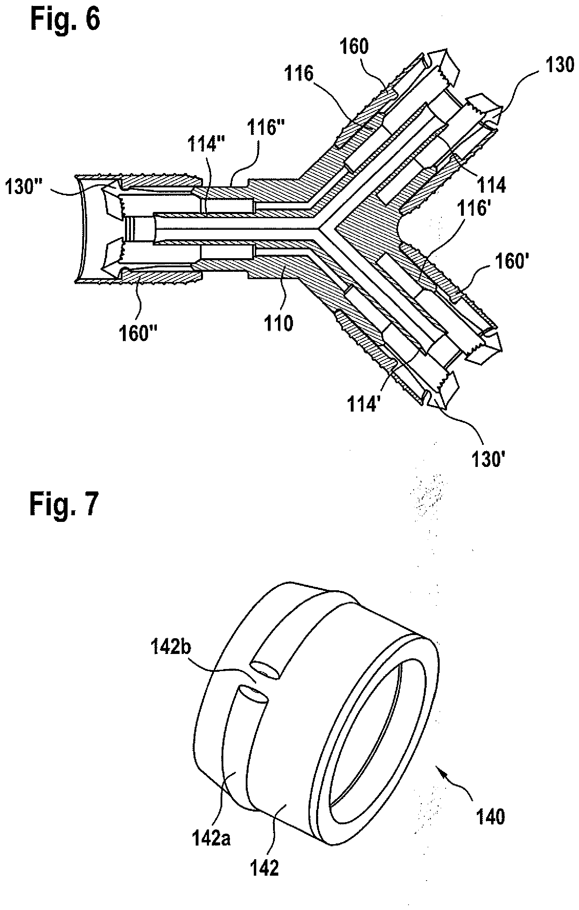

Fig. 7 veranschaulicht eine Ausführungsform, bei der die Wulst 142a nicht vollständig umlaufend ausgebildet ist, sondern zwei diametral gegenüberliegende Unterbrechungen bzw. Öffnungen 142b aufweist. Dies erlaubt den Durchtritt des Sterilisationsgases bei der Ethylendioxid(EO)-Sterilisation.

Im Rahmen der Erfindung ist der Einsatz eines starren Hülsenkörpers nicht zwingend erforderlich. Die Dichtung kann auch anders eingebracht sein. Sie kann beispielsweise geklebt, direkt eingespritzt oder in Form einer handelsüblichen Dichtung verwirklicht sein. Ferner ist es auch nicht zwingend erforderlich, daß die Dichtlamellen verformbar sind. Es reicht aus, wenn der Schlauch nachgiebig ist. Dann bildet der Schlauch den Dichtkörper, welcher sich an eine starre Struktur der Dichthülse anschmiegt. Die Einschubtiefe der Dichthülsen 140 ist durch einen Anschlag der Kammerhülsen begrenzt. In diesem Bereich übergreift der die Dichtlamellen aufweisende Dichtkörper die Stirnfläche der starren Dichthülse 142, um so eine stirnseitige Abdichtung des Ringspalts zwischen Anschlussstutzen 114 und Kammerhülsen 116 zu ermöglichen.

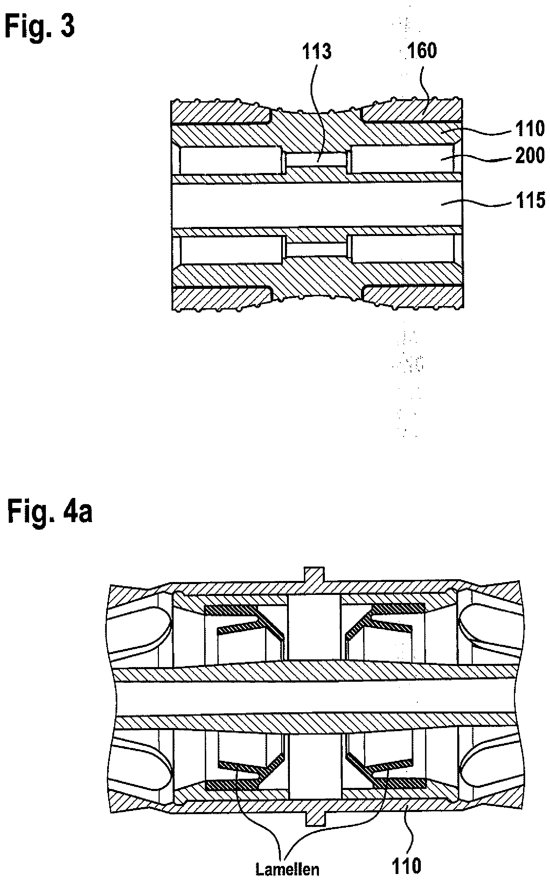

Zwischen den die Einschubtiefe des Schlauchs in den den Ringspalt begrenzenden Anschlägen und dem umlaufenden Bund 112 ist bei eingeschobenem Schlauch eine Verteilerkammer 200 gebildet. Die beidseitig des Bunds 112 gebildeten Verteilerkammern 200 sind über Kanäle 113 (vgl. Fig. 3) miteinander verbunden. Es ist so möglich, die radial versetzt angeordneten Zusatzlumen 30 der in die Ringspalte eingeführten Schlauchmäntel 12 unabhängig von der Drehstellung der Mäntel bezüglich der Verbindungsvorrichtung miteinander zu verbinden, wobei eine Abdichtung der radial versetzt angeordneten Zusatzlumen bezüglich dem zentralen Lumen 20 mit Hilfe der Anschlussstutzen 114 ermöglicht wird. Die Anschlussstutzen 114 werden beim Anschließen der Schläuche 10 in die zentralen Lumen 20 der Schläuche eingeführt. Die äußeren Begrenzungsflächen der Anschlussstutzen 114 erweitern sich ausgehend von den dem Bund 112 abgewandten Enden in Richtung auf den Bund, so daß das zentrale Lumen 20 bei Einführen der Anschlussstutzen 114 aufgeweitet und ein dichter Sitz der Schläuche 10 auf den Anschlussstutzen 114 erreicht wird, wobei gleichzeitig eine Abdichtung durch Anlage der Dichtlamellen 144 an der äußeren Begrenzungsfläche des Mantels 12 erfolgt.

An den Kammerhülsen 116 sind an deren dem Bund 112 abgewandten Enden Klemmzungen 130 angeordnet, die über Materialschwächungen 132 an die Kammerhülsen 116 angesetzt sind. Die Kammerhülsen 116 und Klemmzungen 130 werden von Klemmhülsen 160 umlaufen. Bei der in Fig. 2 dargestellten Ausführungsform der Erfindung weisen die Klemmzungen 130 an ihren den Kammerhülsen 116 abgewandten Enden die Klemmzunge 130 radial erweiternde Verdickungen 136 auf. Zusätzlich sind radial innenliegend die Klemmzungen 130 radial nach innen erweiternde Klemmzähne 138 vorgesehen. Die Klemmhülsen 160 sind auf den inneren Begrenzungsflächen mit radial innenliegenden, die lichte Weite der Klemmhülsen 160 verringernden Klemmringen 162 ausgestattet. Wenn die Klemmhülsen 160 ausgehend von der in Fig. 2 dargestellten Freigabestellung, die das Einführen des mehrlumigen Schlauchs in die Ringspalte zwischen Anschlussstutzen 114 und Kammerhülse 116 ermöglicht, von dem Bund 112 weg verschoben werden, gelangen die Klemmringe 162 in Anlage an die Verdickung 136 und drücken die Klemmzunge 130 insgesamt radial nach innen, wobei die Verlagerung der Klemmzungen 130 durch die Sollbiegestellen 132 im Übergangsbereich zwischen Klemmzungen 130 und Kammerhülsen 160 erleichtert wird.

Im Verlauf der axialen Bewegung der Klemmhülse 160 bezüglich der Klemmzunge 130 gleitet der Klemmring 162 über den Scheitel der Verdickung 136, so daß eine Rückstellbewegung in die in Fig. 2 dargestellte Freigabestellung aus der so erreichten Sicherungsstellung nur unter Überwindung einer Anschubkraft möglich ist. Bei Erreichen der Sicherungsstellung gelangen radial innenliegende Anschlagstege 164 der Klemmhülse 160 in Anlage an radial außenliegende Anschlagstege 134 der Kammerhülse 116, so daß das Erreichen der Sicherungsstellung akustisch bestätigt wird. In der Sicherungsstellung greifen die Klemmzähne 138 in die äußeren Begrenzungsfläche des Schlauchmantels 12 ein, so daß dieser vor einem Ablösen von der Anschlussvorrichtung 100 gesichert ist. Wie in Fig. 2 erkennbar, sind die Klemmhülsen auch in der Freigabestellung in einem durch den Bund 112 mit radialer Erweiterung definierten axialen Abstand voneinander angeordnet. Das erleichtert die separate Betätigung der Klemmhülsen 160. Der Bund 112 weist an seiner äußeren Begrenzungsfläche eine Vertiefung auf, welche den Zugriff auf den Grundkörper erleichtert.

Wie in Fig. 2 weiter zu erkennen ist, sind die Anschlussstutzen 114 des Grundkörpers 110 in unterschiedlichen Längen ausgeführt. Dadurch wird erreicht, daß die zum Ablösen der Schläuche von den Anschlussstutzen benötigten Kräfte ungleich sind. Die Klemmhülsen können stirnseitig abgerundet ausgeführt sein, um so ein Verhaken mit anderen Gegenständen zu vermeiden. Dagegen sind in alternativen Ausführungsformen die Längen der Anschlußstutzen 114 gleich. An den äußeren Begrenzungsflächen der Klemmhülsen können Markierungen angebracht sein, welche die möglichen Bewegungsrichtungen anzeigen. Diese Markierungen können beispielsweise in Form von axialen Doppelpfeilen, Beschriftungen (auf/zu) und/oder Symbolen (Vorhängeschloß usw.) ausgeführt sein. Die Markierungen können als erhabene/vertiefte und ggf. aufgerauhte/polierte Flächenbereiche ausgeführt oder einfach aufgemalt, aufgeklebt, usw. sein, um so die Bedienung der Anschlussvorrichtung zu erleichtern. Die Flächenbereiche der Klemmhülsen können aufgerauht sein und/oder Rillen, Ringe, Strukturierungen enthalten etc. (oder auch ohne Markierung), um ein Abrutschen der Finger bei der Bedienung zu vermeiden.

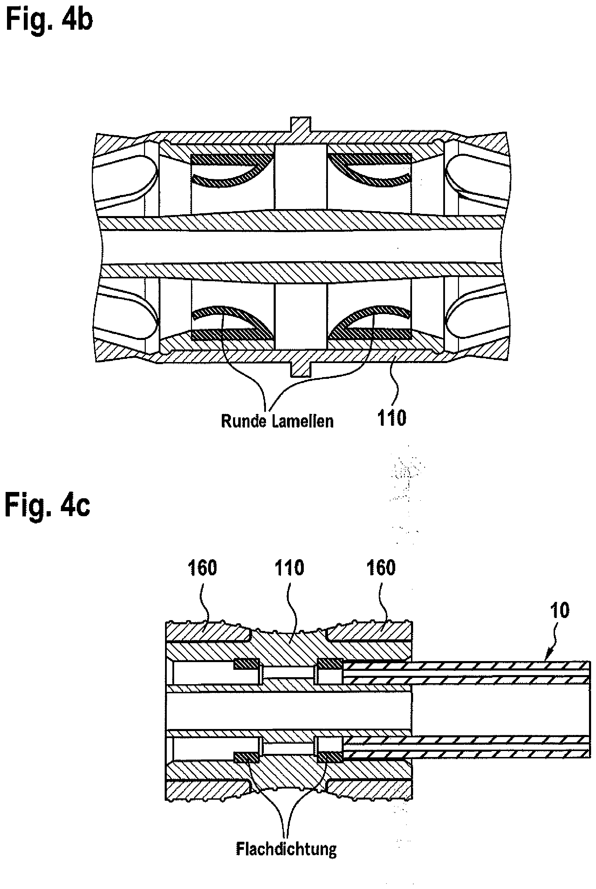

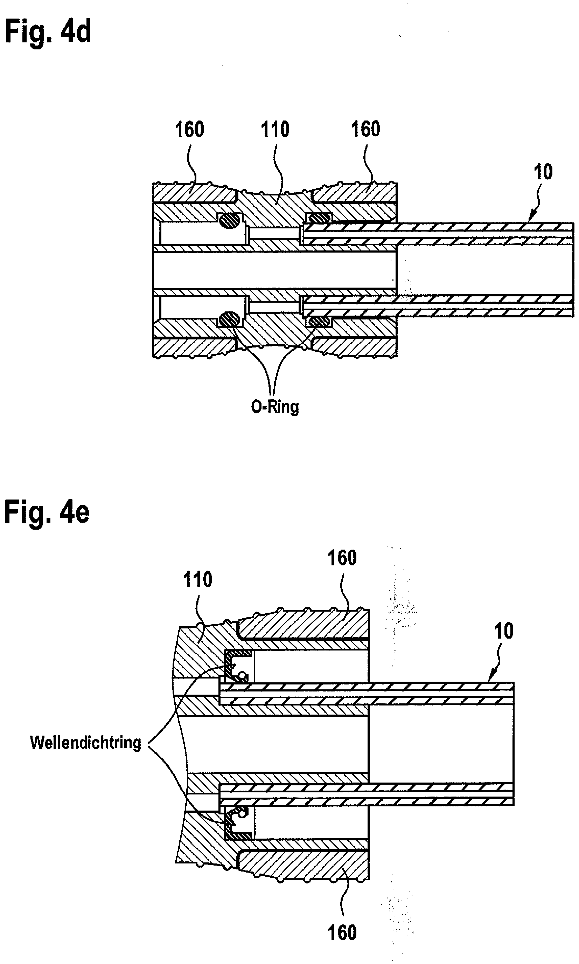

In Fig. 4 sind Variationen möglicher Abdichtungen zwischen mehrlumigem Schlauch und Grundkörper einer erfindungsgemäßen Anschlussvorrichtung dargestellt. Dabei zeigt Fig. 4a eine mehrseitige Lamellendichtung, Fig. 4b eine Dichtung mit runder Lamelle, Fig. 4c eine stirnseitige Abdichtung außerhalb der radial versetzt angeordneten Zusatzlumen zwischen den Stirnflächen der Schläuche und Bund 112. Fig. 4d zeigt eine Abdichtung über als O-Ringe ausgeführte Dichtringe. Schließlich kann gemäß Fig. 4e auch ein Radialwellendichtring eingesetzt werden.

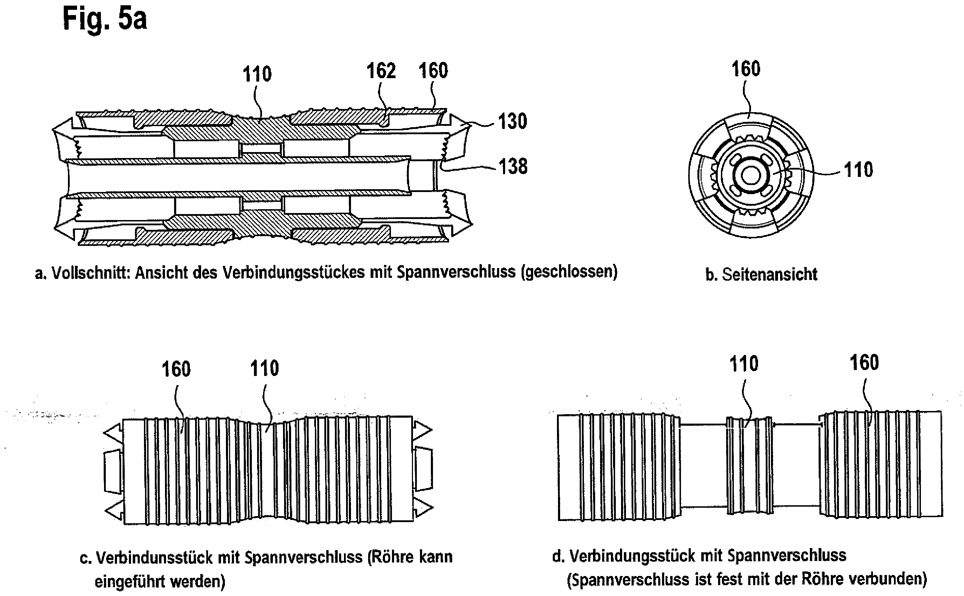

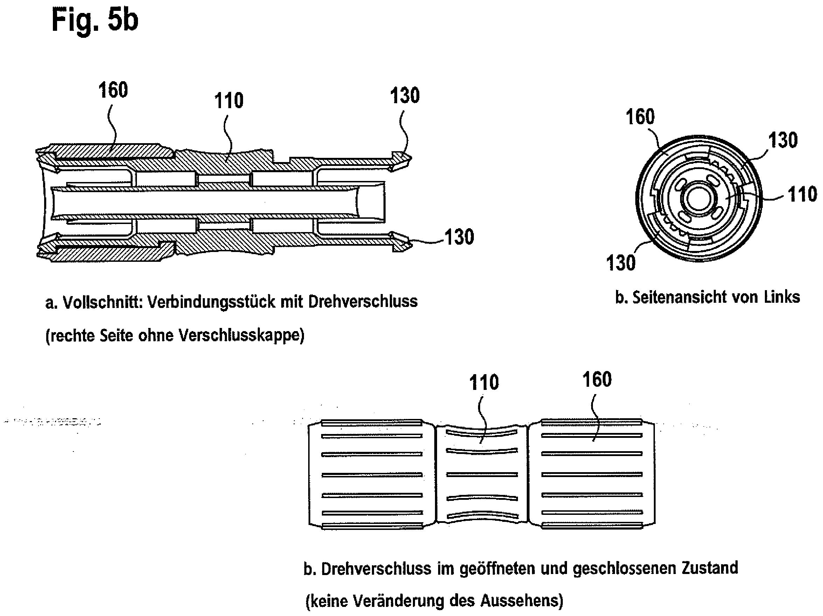

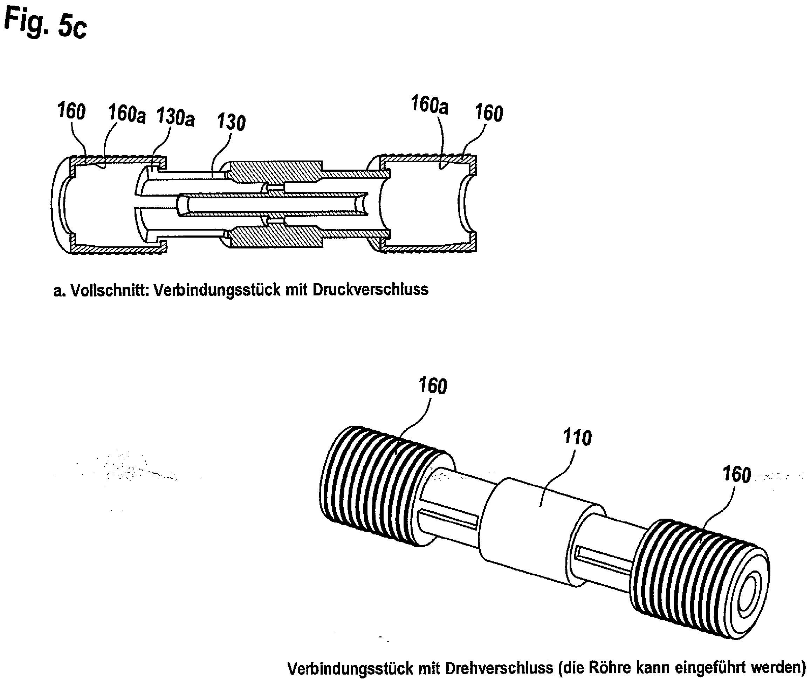

In Fig. 5 sind Variationen von Sicherungseinrichtungen erfindungsgemäßer Anschlussvorrichtungen dargestellt. Dabei entspricht Fig. 5a im Wesentlichen der Sicherungseinrichtung gemäß Fig. 2, wobei ein radial innenliegender Klemmring 162 bei Verstellung der Klemmhülse 160 von der Freigabestellung in die Sicherungsstellung längs sich radial erweiternder Klemmrampen der Klemmzungen 130 gleitet und die Klemmzungen 130 so radial nach innen versetzt, wobei die Klemmzähne 138 in Eingriff mit der äußeren Begrenzungsfläche des Schlauchmantels 12 gelangen. Bei der in Fig. 5a dargestellten Ausführungsform sind insgesamt vier in Umfangsrichtung voneinander beabstandete Klemmzungen 130 vorgesehen, die mit Hilfe der axialen Bewegung der Klemmhülse 160 gegen die äußere Begrenzungsfläche des Schlauchmantels 12 gedrängt werden können. Dabei werden die Klemmhülsen 160 auseinandergezogen, wie sich aus einer vergleichenden Betrachtung der unterschiedlichen Darstellungen in Fig. 5a ergibt. Rechts unten ist die Sicherungsstellung der Sicherungseinrichtung dargestellt.

Bei der in Fig. 5b dargestellten Ausführungsform der Erfindung wird die Sicherungsstellung durch Verdrehen der Klemmhülse erreicht. Dazu sind im Bereich der inneren Begrenzungsfläche der Klemmhülse sich in Umfangsrichtung erstreckende Klemmrampen vorgesehen, die durch Drehen der Klemmhülse eine radiale Verlagerung der Klemmzungen 130 bewirken. Ein Vorteil dieser Ausführungsform der Erfindung ist der reduzierte Platzbedarf.

Schließlich können die Klemmzungen bei der Ausführungsform gemäß Fig. 5c durch axiales Zusammendrücken der Klemmhülsen 160 radial nach innen versetzt werden. Hierzu sind an der inneren Begrenzungsfläche der Klemmhülsen 160 entsprechende Klemmrampen 160a vorgesehen, die mit Verdickungen 130a an den äußeren Begrenzungsflächen der Klemmzungen 130 zusammenwirken.

Die Anschlussvorrichtung gemäß Fig. 6 ist für insgesamt drei mehrlumige Schläuche gedacht. Dazu weist die Anschlussvorrichtung gemäß Fig. 6 insgesamt drei Anschlussstutzen 114, 114', 114" auf, die jeweils von einer Kammerhülse 116, 116', 116" umlaufen sind, so daß insgesamt drei Ringkammern zum Einführen von Schlauchmänteln mehrlumiger Schläuche zur Verfügung stehen. Die zentralen Lumen der Schläuche können über die Anschlussstutzen durchsetzende Bohrungen miteinander verbunden werden. Die radial versetzt angeordneten Zusatzlumen können über entsprechende Kanäle in dem Grundkörper 110 verbunden werden. Jedem Anschlussstutzen der in Fig. 6 dargestellten Ausführungsform der Erfindung sind Klemmzungen 130, 130', 130" und Klemmhülsen 160, 160', 160" der anhand der in Fig. 2 bis 4 dargestellten Art zugeordnet. Einer der Anschlussstutzen 160" der in Fig. 6 dargestellten Anschlussvorrichtung ist kürzer ausgeführt als die beiden übrigen Anschlussstutzen 160, 160'. Dieser Anschlussstutzen dient beispielsweise zur vakuumtherapiegeräteseitigen Verbindung eines mehrlumigen Schlauchs. Die Verbindung kann einfacher gelöst werden als die Verbindung der übrigen auf die anderen Anschlussstutzen aufgeschobenen mehrlumigen Schläuche, weil bei diesen anderen Schläuchen die Anschlussstutzen ein Widerlager für die Klemmzungen bildet, so daß die Klemmkraft verstärkt werden kann. So kann eine "Sollbruchstelle" im Bereich dieses Anschlussstutzens bereitgestellt werden.

Die Erfindung ist nicht auf die anhand der Zeichnung erläuterten Ausführungsformen der Erfindung beschränkt. Erfindungsgemäße Anschlussvorrichtungen können auch zum direkten Anschließen eines mehrlumigen Schlauchs an ein Vakuumtherapiegerät eingesetzt werden. Ferner können sie zum Verbinden von vier oder mehr mehrlumigen Schläuchen eingesetzt werden. Dabei kann eine beliebige Anzahl von Zusatzlumen zum Einsatz kommen, die über Verteilerkammern verbunden werden.

## Patentansprüche

1. Vorrichtung zum Anschließen von ein zentrales koaxial zur Leitungsachse verlaufendes Lumen und mindestens ein radial versetzt angeordnetes Zusatzlumen aufweisenden mehrlumigen Leitungen für den medizinischen Bereich, insbesondere für die Wundversorgung im Bereich der Unterdrucktherapie, mit einem zum dichten und vorzugsweise lösbaren Anschließen des zentralen Lumens und des mindestens einem Zusatzlumens ausgelegten, vorzugsweise einstückigen Grundkörper (110), wobei der Grundkörper (110) zur Bereitstellung einer Verteilerkammer (200) für mindestens eines, vorzugsweise zwei, drei oder mehr der darin mündenden Zusatzlumen der angeschlossenen Leitung ausgelegt ist und mindestens einen Kanal (113) zum Ab- bzw. Einleiten eines Fluids aus der bzw. in die Verteilerkammer aufweist, wobei die Verteilerkammer bei angeschlossener Leitung derart fluiddicht bezüglich mindestens eines Leitungslumens abgedichtet ist, daß ein Fluidaustausch zwischen der Verteilerkammer und diesem Leitungslumen verhindert ist, wobei der Grundkörper (110) mindestens einen zum Anschließen eines Leitungslumens, insbesondere zum Einführen in mindestens ein Leitungslumen ausgelegten Anschlussstutzen (114) aufweist, die Verteilerkammer (200) den Anschlussstutzen (114) zumindest teilweise umläuft, der Kanal (113) in einem den Anschlussstutzen zumindest teilweise umlaufenden und vorzugsweise einstückig damit ausgeführten Bund (112) gebildet ist, wobei sich der Kanal vorzugsweise etwa parallel zum Anschlussstutzen (114) erstreckt und die Verteilerkammer (200) von einer sich ausgehend von dem Bund (112) etwa parallel zum Anschlussstutzen (114) erstreckenden insbesondere einstückig mit dem Bund (112) ausgeführten Kammerhülse (116) begrenzt ist, wobei zwischen Anschlussstutzen (114) und Kammerhülse (116) ein Ringspalt zum Aufnehmen eines mit mindestens einem Lumen durchsetzten Mantels (12) einer mehrlumigen Leitung (10) ausgelegt ist, **gekennzeichnet durch** eine zwischen einer das Anschließen der mehrlumigen Leitung ermöglichenden Freigabestellung und einer die angeschlossene Leitung an dem Grundkörper sichernde, vorzugsweise akustisch bestätigbare Sicherungsstellung verstellbaren Sicherungseinrichtung (130, 160), wobei die Sicherungseinrichtung eine koaxial zur Kammerhülse (116) verlaufende Klemmhülse (160) aufweist, die zwischen der Freigabestellung und der Sicherungsstellung verdreh- und/oder axial verschiebbar ist, wobei dem Grundkörper (110) mindestens ein Klemmkörper (130) zugeordnet ist, der in der Sicherungsstellung gegen eine äußere Begrenzungsfläche der Leitung gedrängt werden kann und der Klemmkörper mindestens eine einstückig mit der Kammerhülse (116) ausgeführte und sich ausgehend davon etwa parallel zur Hülsenachse erstreckende Klemmzunge (130) aufweist, deren der Kammerhülse (116) abgewandtes Ende durch Verstellen der Sicherungseinrichtung in die Sicherungsstellung radial nach innen in Richtung auf die Hülsenachse gedrängt wird.

2. Vorrichtung nach Anspruch 1, **gekennzeichnet durch** einen das Einführen des Mantels (12) in den Ringspalt begrenzenden und in einem axialen Abstand von dem Bund (112) angeordneten, vorzugsweise als radialer Absatz an dem Anschlussstutzen und/oder der Kammerhülse ausgeführten Anschlag (120).

3. Vorrichtung nach Anspruch 1 oder 2, **gekennzeichnet durch** eine bei Einführen des Mantels in den Ringspalt in Anlage an eine Stirnfläche des Mantels gelangende Dichtungsanordnung (140).

4. Vorrichtung nach Anspruch 2 oder 3, **gekennzeichnet durch** eine bei Einführen des Mantels in den Ringspalt in Anlage an eine die Leitungsachse umlaufende äußere Begrenzungsfläche des Mantels gelangende weitere Dichtungsanordnung (140).

5. Vorrichtung nach Anspruch 4, **dadurch gekennzeichnet, daß** die weitere Dichtungsanordnung (140) eine in dem Ringspalt aufgenommene Dichthülse (140) mit mindestens einer, vorzugsweise zwei, drei oder mehr axial voneinander beabstandete und die Hülsenachse umlaufende und in Anlage an die äußere Begrenzungsfläche des Mantels gelangende Dichtlippen (144) aufweist.

6. Vorrichtung nach einem der vorhergehenden Ansprüche, **dadurch gekennzeichnet, daß** der Grundkörper (110) zum Bereitstellen von zwei, drei oder mehr Verteilerkammern ausgelegt ist, von denen mindestens zwei vorzugsweise über mindestens einen Kanal miteinander verbunden sind.

7. Vorrichtung nach Anspruch 6, **dadurch gekennzeichnet, daß** zwei, drei oder mehr der Verteilerkammern einen zum Anschließen eines Leitungslumens, insbesondere zum Einführen in mindestens ein Leitungslumen ausgelegten, ggf. Y-förmig angeordneten Anschlussstutzen (114) zumindest teilweise umlaufen, wobei vorzugsweise mindestens zwei Verteilerkammern jeweils mindestens eine Dichtungsanordnung (140) und/oder mindestens eine Sicherungsanordnung (130, 160) zugeordnet ist.

8. Vorrichtung nach Anspruch 7, **dadurch gekennzeichnet, daß** sich die zum Lösen der Leitungen von dem Grundkörper (112) benötigten Kräfte bei in die Sicherungsstellung verstellten Sicherungseinrichtungen für mindestens zwei Sicherungseinrichtungen voneinander unterscheiden.

9. Behandlungskit mit mindestens einer Anschlussvorrichtung (100) nach einem der vorhergehenden Ansprüche und mindestens einer daran anschließbaren mehrlumigen Leitung (10).

10. Behandlungskit nach Anspruch 9, **dadurch gekennzeichnet, daß** die Leitung (10) einen Schlauch mit einem von einem Schlauchmantel (12) begrenzten zentralen Lumen (20) und mindestens einem den Schlauchmantel (12) in einer parallel zur Schlauchachse und parallel zum zentralen Lumen (20) verlaufenden Richtung durchsetzenden Zusatzlumen (30) aufweist, wobei das mindestens eine Zusatzlumen (30) nach Anschluss an die Anschlussvorrichtung (100) in die Verteilerkammer mündet und/oder der Anschlussstutzen (114) in das zentrale Lumen (20) einführbar ist.

## Claims

1. A device for connecting multi-lumen lines, having a central lumen extending coaxially to the line axis and at least one radially offset additional lumen for the medical field, in particular for wound care in the field of vacuum therapy, to a preferably one-piece basic body (110) designed for tight and preferably releasable connection of the central lumen and the at least one additional lumen, wherein the basic body (110) is designed to provide a distributor chamber (200) for at least one, preferably two, three or more additional lumens of the line connected thereto that open into the distributor chamber, and includes at least one channel (113) for removing a fluid from or introducing a fluid to the distributor chamber, wherein when a line is connected, the distributor chamber is sealed off in fluid-tight fashion from at least one line lumen such that fluid exchange between the distributor chamber and this line lumen is prevented, wherein the basic body (110) has at least one connection fitting (114) for connecting a line lumen, in particular for insertion into at least one line lumen, the distributor chamber (200) surrounds at least partially the connection fitting (114), the channel (113) is formed in a collar (112) surrounding at least partially the connection fitting and is preferably fashioned as a single piece together with the connection fitting, wherein the channel preferably extends approximately parallel to the connection fitting (114) and the distributor chamber (200) is confined by a chamber bushing (116) starting from the collar (112) and extending approximately parallel to the connection fitting (114), which bushing is in particular fashioned as a single piece together with the collar (112), wherein an annular gap is formed between the connection fitting (114) and chamber bushing (116) for receiving a sheath (12) of a multilumen line (10) through which at least one lumen extends, **characterized by** a securing device (130, 160) that is movable between a release position, which enables the connection of the multi-lumen line, and a securing position, which secures the connected line to the basic body and can preferably be confirmed acoustically, wherein the securing device has a clamping bushing (160) extending coaxially to the chamber bushing (116) and rotatable and/or axially translatable between the release position and securing position, wherein at least one clamping body (130) is associated with the basic body (110), which clamping body can be pushed against an outer boundary face of the line in the securing position and has at least one clamping tongue (130) which is fashioned as a single piece together with the chamber bushing (116) and which, starting from the clamping bushing, extends approximately parallel to the bushing axis, and whose end facing away from the chamber bushing (116) is pushed radially inwards towards the bushing axis when the securing device is moved to the securing position.

2. The device according to claim 1, **characterised by** a stop (120), placed at an axial distance from the collar (112) and preferably designed as a radial shoulder on the connection fitting and/or chamber bushing, which limits the insertion of the sheath (12) into the annular gap.

3. The device according to claim 1 or 2, **characterised by** a sealing arrangement (140) that comes into contact with an end face of the sheath when the sheath is inserted into the annular gap.

4. The device according to claim 2 or 3, **characterised by** a further sealing arrangement (140) that, when the sheath is inserted into the annular gap, comes into contact with an outer boundary face of the sheath surrounding the line axis.

5. The device according to claim 4, **characterised in that** the further sealing arrangement (140) includes a sealing shell (140), received in the annular gap, comprising at least one, preferably two, three or more sealing lips (144) spaced apart from each other axially, surrounding the shell axis and coming into contact with the outer boundary face of the sheath.

6. The device according to one of the preceding claims, **characterised in that** the basic body (110) is designed to provide two, three or more distributor chambers, at least two of which are preferably connected to each other via at least one channel.

7. The device according to claim 6, **characterised in that** two, three or more of the distributor chambers surround at least partially a connection fitting (114) which is designed for connecting a line lumen, in particular for insertion into at least one line lumen, and where appropriate is arranged in a Y-shape, wherein at least one sealing arrangement and/or at least one securing arrangement (130, 160) are associated with preferably at least two distributor chambers (140) respectively.

8. The device according to claim 7, **characterised in that** the forces required to release the lines from the basic body (112) when the securing devices are in the securing position differ between at least two securing devices .

9. A care kit comprising at least one connection device (100) according to one of the preceding claims and at least one multi-lumen line (10) which can be connected to this connection device.

10. The care kit according to claim 9, **characterised in that** the line (10) includes a tube comprising a central lumen (20) confined by a tube sheath (12) and comprising at least one additional lumen (30) extending through the tube sheath (12) in a direction running parallel to the tube axis and parallel to the central lumen (20), wherein the at least one additional lumen (30) opens into the distributor chamber once connected to the connection device (100) and/or the connection fitting (114) can be inserted into the central lumen (20).

## Revendications

1. Dispositif permettant de raccorder des conduites à plusieurs lumières, c'est-à-dire une lumière centrale coaxiale à l'axe de la conduite et au moins une lumière supplémentaire à décalage radial, destinées au domaine médical, notamment pour le soin des plaies dans le domaine de la thérapie par pression négative, à un corps de base (110) de préférence unitaire destiné au raccordement étanche et de préférence amovible de la lumière centrale et de l'au moins une lumière supplémentaire, ledit corps de base (110) étant conçu pour fournir une chambre de distribution (200) pour au moins une, et de préférence deux, trois ou davantage de lumières supplémentaires de la conduite raccordée qui y débouchent, et présentant au moins un canal (113) permettant d'évacuer un fluide hors de ladite chambre de distribution ou de l'y introduire, ladite chambre de distribution étant étanche aux fluides par rapport à au moins une des lumières de la conduite, lorsqu'une conduite est raccordée, de façon à empêcher tout échange de fluide entre la chambre de distribution et cette lumière de la conduite, ledit corps de base (110) présentant au moins une tubulure de raccordement (114) conçue pour raccorder une lumière de la conduite, notamment pour être introduite dans au moins une lumière de la conduite, ladite chambre de distribution (200) entourant au moins partiellement la tubulure de raccordement (114), ledit canal (113) étant formé dans un flasque (112) qui entoure au moins partiellement ladite tubulure de raccordement et est de préférence réalisé d'un seul tenant avec cette dernière, ledit canal s'étendant de préférence approximativement parallèlement à la tubulure de raccordement (114) et ladite chambre de distribution (200) étant délimitée par un manchon de chambre (116) qui s'étend à partir du flasque (112) à peu près parallèlement à la tubulure de raccordement (114) et qui est notamment réalisé d'un seul tenant avec ledit flasque (112), étant entendu qu'un espace annulaire destiné à recevoir une enveloppe (12), traversée par au moins une lumière, d'une conduite à plusieurs lumières (10) est conçu entre la tubulure de raccordement (114) et le manchon de chambre (116) ; **caractérisé par** un dispositif de fixation (130, 160) susceptible de basculer entre une position de libération permettant le raccordement de la conduite à plusieurs lumières et une position de fixation, de préférence à rétroaction sonore, qui fixe au corps de base ladite conduite raccordée, ledit dispositif de fixation présentant un manchon de serrage (160) coaxial au manchon de chambre (116) et susceptible de tourner et/ou d'être translaté axialement entre la position de libération et la position de fixation, au moins un corps de serrage (130) étant associé au corps de base (110), lequel, en position de fixation, peut être pressé contre une surface de délimitation extérieure de la conduite, ledit corps de serrage présentant au moins une languette de serrage (130) conçue d'un seul tenant avec le manchon de chambre (116) et s'étendant de manière approximativement parallèle à l'axe du manchon à partir de celui-ci, et dont l'extrémité opposée au manchon de chambre (116) est pressée radialement vers l'intérieur en direction de l'axe du manchon lorsque le dispositif de fixation passe en position de fixation.

2. Dispositif selon la revendication 1, **caractérisé par** une butée (120) qui limite l'introduction de l'enveloppe (12) dans l'espace annulaire et qui est située axialement à distance du flasque (112) en étant de préférence conçue sous la forme d'un épaulement radial sur la tubulure de raccordement et/ou le manchon de chambre.

3. Dispositif selon la revendication 1 ou 2, **caractérisé par** un dispositif d'étanchéité (140) qui vient au contact d'une face terminale de ladite enveloppe lorsque cette dernière est introduite dans l'espace annulaire.

4. Dispositif selon la revendication 2 ou 3, **caractérisé par** un dispositif d'étanchéité (140) supplémentaire qui vient au contact d'une surface de délimitation extérieure de l'enveloppe qui entoure l'axe de la conduite lorsque l'enveloppe est introduite dans l'espace annulaire.

5. Dispositif selon la revendication 4, **caractérisé en ce que** le dispositif d'étanchéité (140) supplémentaire (140) présente un manchon d'étanchéité (140) logeant dans l'espace annulaire et doté d'au moins une et de préférence deux, trois ou davantage de lèvres d'étanchéité (144) espacées axialement les unes des autres en entourant l'axe du manchon et en contact avec la surface de délimitation extérieure de l'enveloppe.

6. Dispositif selon l'une des revendications précédentes, **caractérisé en ce que** le corps de base (110) est conçu pour fournir deux, trois ou davantage de chambres de distribution, dont au moins deux sont de préférence reliées entre elles par au moins un canal.

7. Dispositif selon la revendication 6, **caractérisé en ce que** deux, trois ou davantage de chambres de distribution entourent au moins partiellement une tubulure de raccordement (114) conçue pour raccorder une lumière de la conduite, notamment pour être introduite dans au moins une lumière de la conduite et éventuellement en forme de Y, au moins un dispositif d'étanchéité (140) et/ou au moins un dispositif de fixation (130, 160) étant de préférence respectivement associés à au moins deux chambres de distribution.

8. Dispositif selon la revendication 7, **caractérisé en ce que** les forces nécessaires pour détacher les conduites du corps de base (112) lorsque les dispositifs de fixation sont en position de fixation sont différentes pour au moins deux dispositifs de fixation.

9. Kit de traitement comprenant au moins un dispositif de raccordement (100) selon l'une des revendications précédentes et au moins une conduite à plusieurs lumières (10) susceptible de s'y raccorder.

10. Kit de traitement selon la revendication 9, **caractérisé en ce que** la conduite (10) comprend un tube doté d'une lumière centrale (20) délimitée par une enveloppe de tube (12) et d'au moins une lumière supplémentaire (30) traversant l'enveloppe de tube (12) dans une direction parallèle à l'axe dudit tube et parallèle à la lumière centrale (20), l'au moins une lumière supplémentaire (30) débouchant dans la chambre de distribution après raccordement au dispositif de raccordement (100) et/ou la tubulure de raccordement (114) pouvant être introduite dans ladite lumière centrale (20).
